# EUROPEAN PATENT SPECIFICATION

(11) **EP 3 119 276 B1**
(45) Date of publication and mention of the grant of the patent: **21.02.2024**
(21) Application number: 15765634.9
(22) Date of filing: 19.03.2015
(51) Int. Cl.: A61B 90/00, A61B 5/06

(54) **SYSTEM FOR USING BODY SURFACE CARDIAC ELECTROGRAM INFORMATION COMBINED WITH INTERNAL INFORMATION TO DELIVER THERAPY**
SYSTEM ZUR VERWENDUNG VON HERZELEKTROGRAMMINFORMATIONEN DER KÖRPEROBERFLÄCHE KOMBINIERT MIT INTERNEN INFORMATIONEN ZUR BEREITSTELLUNG EINER THERAPIE
SYSTÈME D'UTILISATION D'INFORMATIONS D'ÉLECTROCARDIOGRAMME DE SURFACE CORPORELLE COMBINÉES À DES INFORMATIONS INTERNES POUR ADMINISTRER UNE THÉRAPIE

(30) Priority: 19.03.2014 US 201461955673 P
(43) Date of publication of application: 25.01.2017
(73) Proprietor: Cardionxt, Inc., Westminster, CO 80021 (US)
(72) Inventor: EDWARDS, Jerome Ranjeev, Erie, Colorado 80516 (US); KESSMAN, Paul, Lakewood, Colorado 80221 (US); NGUYEN, Bao, Erie, Colorado 80516 (US)
(74) Representative: Cooley (UK) LLP
(86) International application number: PCT/US2015/021435
(87) International publication number: WO 2015/143136

(56) References cited:
- US-A1- 2004 015 194
- US-A1- 2006 173 269
- US-A1- 2006 173 269
- US-A1- 2008 058 657
- US-A1- 2011 288 605
- US-A1- 2013 267 835
- US-A1- 2013 274 593

## Description

### Priority

This application claims priority, under 35 U.S.C. § 119(e), to commonly owned and assigned U.S. Provisional Patent Application No. 61/955,673, entitled "System and Methods for Integrating Cardiac Electrical Maps and Intraprocedural Information," filed on March 19, 2014.

### Field of the Invention

The invention generally relates to systems and methods (a method is not claimed) that aid physicians in performing surgical procedures on patients. More specifically, the invention relates to systems and methods for non-invasively mapping the electrical activity of the heart and identifying sources of arrhythmia using electrodes on the patient's external body surface, projecting that information onto a computer imaged three-dimensional or four-dimensional model of the heart, and co-registering the model based on cardiac activity information with a position localization system which can be used to accurately navigate instruments during a procedure with respect to the sources of arrhythmia for treatment of the patient, and further augmenting the cardiac activity information with real-time intracardiac recordings from instruments navigated during the surgical procedure.

### Background of the Invention

There exist very complex cardiac arrhythmias such as Atrial Fibrillation that are extremely hard to deconstruct to a source with conventional intracardiac catheters and traditional twelve-lead electrocardiogram readings. There are high resolution cardiac electrogram processing techniques that utilize large numbers of sampling electrodes spread all over a patient's thorax along with imaging techniques such as computerized tomography or magnetic resonance imaging to create models of the heart and project electrical activity at the body surface onto these imaging models. Ultimately, to make use of this high resolution body surface cardiac electrogram information for treating patients, the information must be presented in a manner in which the surgeon can process that information during a surgery, identify sources of the arrhythmia, translate that arrhythmia source information to anatomic information that they are able to manipulate and then deliver therapy to that source to treat the patient. Further, the electrical activity of the heart is constantly changing and measurements acquired from outside the body must be augmented with measurements acquired from inside the body to best highlight potential anatomic source regions of the arrhythmia. This must all be performed in a stable and consistent way, over heart beat and respiration cycles, so that the surgeon can trust the information before they deliver therapy to a particular region of the heart. Serious complications, such as sudden cardiac arrest, stroke, atrio-esophageal fistula and perforation can occur if therapy is delivered internally inaccurate based on the body surface cardiac electrical information.

Known systems are disclosed in US 2004/015194 A1, US 2006/173269 A1, US 2013/274593 A1, and US 2008/058657 A1.

Therefore, a practical need exists to have an accurate way of delivering body surface cardiac electrical information to a surgeon during a procedure in which they are manipulating instruments inside the body to diagnose the source of an arrhythmia and deliver therapy to that source.

### Summary of the Invention

Embodiments of the invention are summarized below. These and other embodiments are described in the Detailed Description. It is to be understood, however, that there is no intention to limit the invention to the forms described herein. One skilled in the art can recognize that there are numerous modifications, equivalents, and alternative constructions that fall within the spirit and scope of the invention as expressed in the claims.

In accordance with the invention, there is provided a system as defined in claim 1. Optional features are defined in the dependent claims.

### Brief Description of the Drawings

Figure 1 illustrates an electrical cardiac activity mapping system and a cardiac instrument position mapping system, according to an embodiment.
Figure 2 illustrates an example of a computer screen output of an electrical cardiac activity mapping system and cardiac instrument position mapping system, according to an embodiment.
Figure 3 illustrates an example of a computer screen output of an electrical cardiac activity mapping system output projected onto a heart model, according to an embodiment.
Figure 4 illustrates an example of a use of 12-Lead EKG to map electrical activity of the heart from the body surface, according to an embodiment.

### Detailed Description

In some embodiments, physicians can utilize multiple independent data components, each data component providing diagnostic clinical data about a patient. For example, a first data component can include an electrocardiogram (EKG) map that can identify an atrial tachycardia. A second data component can include an instrument position map that identifies the positions of instruments inside the heart based on a position sensing system that can aid the physician in manipulating instruments during an electrophysiology study. The electrocardiogram map can help localize the region within the atria identified as the source of ectopic activity driving a cardiac arrythmia. Catheters can be navigated, using the instrument position map, to that region of the heart. Further information can be gathered from that region of the atrium, such as, for example, local activation and voltage, to identify the specific ectopic pathologic source to treat.

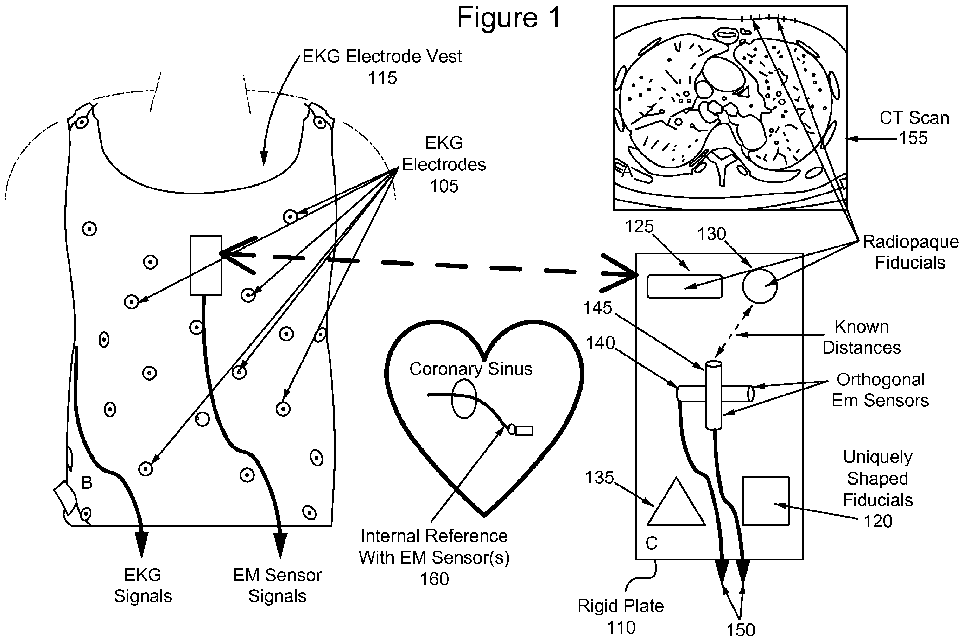

As shown in Figure 1, multiple electrodes 105 can be placed on the body surface of a patient (i.e., body surface electrodes). In some embodiments, the multiple electrodes 105 can be included in an EKG electrode vest 115 that can be worn by the patient, as seen in Figure 1. In other embodiments, the multiple electrodes 105 can be individually placed on the surface of the patient's body. In yet other embodiments, the multiple electrodes 105 can be included in any other suitable system that allows the medical professional to place an array of electrodes 105 on the patient, such as, for example, a blanket that can be placed over the patient, without requiring the patient to wear it like a garment.

Referring again to Figure 1, the EKG electrode vest 115 can be any suitable, non-conductive material that is radiotranslucent, such as, for example, cotton, polyester, or rayon. Radiotranslucent materials do not appear in the final image taken by medical imaging equipment, such as, for example, magnetic resonance imaging (MRI), computed tomography (CT) scans, X-Rays, and so forth.

The multiple electrodes 105 within the electrode vest 115 can be made from any suitable conductive material, such as, for example, silver-chloride, platinum, copper, or gold. The electrode array can include any number of electrodes 105. As shown in Figure 1, the electrodes 105 can be disposed in a pattern such that each electrode 105 is substantially the same distance from each surrounding electrode 105. In other embodiments, the electrodes 105 can be disposed in any other pattern and/or can be disposed such that the electrodes 105 are different distances from each surrounding electrode 105.

Also on the EKG electrode vest 115, as shown in Figure 1, can be a rigid plate 110. The rigid plate 110 can be any suitable, radiotranslucent, rigid material including, for example, plastic. In some embodiments, the rigid plate 110 can be any non-ferrous material such that the rigid plate 110 can be used in MRI environments. In some embodiments, the rigid plate 110 can include an adhesive on the surface of the rigid plate 110 for securing the rigid plate 110 to the patient's body surface.

While shown in Figure 1 as part of the electrode vest 115, in some embodiments, the multiple electrodes 105 and/or rigid plate 110 can be placed on the patient's body individually, without being part of a garment. In other embodiments, the rigid plate 110 can be included in any other suitable system that can allow a medical professional to place the rigid plate and/or multiple electrodes 105 on the patient without being part of a garment, such as, for example, a blanket.

The rigid plate 110 can include radiopaque fiducials (i.e., markers) 120, 125, 130, 135 such that the fiducials 120, 125, 130, 135 do appear in the final image taken by medical imaging equipment. Radiopaque fiducials 120, 125, 130, 135 can be any suitable material such that the fiducial 120, 125, 130, 135 will enhance in, for example, MRI, CT, or X-Ray imagery, such as, for example, steel or copper. In some embodiments, the fiducial 120, 125, 130, 135 can be soaked in a contrast medium such as, for example, gadolinium, such that it will enhance in MRI imaging. The rigid plate 110 and associated fiducials 120, 125, 130, 135 can be designed with a correct consistency of radiopacity to enhance in cone-beam-CT or other forms of intraprocedural X-Ray imaging. The radiopaque fiducials 120, 125, 130, 135 can be any suitable shape including, for example, circular, donut, triangular, square, and/or rectangular. The fiducials 120, 125, 130, 135 are also sometimes called imaging fiducials because they appear in imaging output.

In some embodiments, one or more fiducials 120, 125, 130, 135 can be electrodes and wiring used to collect body surface electrogram information (i.e., cardiac electrical activity information). In some embodiments, one or more fiducials 120, 125, 130, 135 can be position sensors to a position tracking system.

In some embodiments, the rigid plate 110 can be radiopaque and the fiducials 120, 125, 130, 135 can be a cutout in the rigid plate such that the rigid plate will appear in the final image taken by a medical imaging system. Such a configuration can allow the unique shape of the fiducial 120, 125, 130, 135 to appear in the image as contrasted with the rigid plate 110. Similarly stated, rather than a radiopaque fiducial 120, 125, 130, 135, the fiducial 120, 125, 130, 135 can be a void in the radiopaque rigid plate 110, which can still be visible in the final image from the medical imaging system (i.e., an imaging fiducial).

In some embodiments, the rigid plate 110 can include cut-outs for standard medical diagnostics placed on patients such as, for example, 12-Lead EKG pads.

Included on the rigid plate 110 can be multiple electromagnetic position sensors 140, 145. The multiple electromagnetic sensors 140, 145 can be any suitable electromagnetic sensor such as, for example, a copper coil winding or a gold coil winding. The electromagnetic sensors 140, 145 can be disposed on the rigid plate 110 orthogonally to one another, as shown in Figure 1. Each electromagnetic sensor 140, 145 can include a lead 150 that can allow the sensor signal to be captured by a position sensing system coupled to the lead. In some embodiments, the rigid plate 110 can include a housing to enable the electromagnetic position sensors 140, 145 to be removably coupled (e.g., clipped in and out) such that the position sensors 140, 145 can be removed and added with repeatability and reproducibility.

The fiducials 120, 125, 130, 135 and electromagnetic sensors 140, 145 on the rigid plate 110 can be disposed such that each is a known distance between the other components. For example, as shown in Figure 1, the circular fiducial 130 is a known distance from the electromagnetic sensor 140.

The multiple electrodes 105 within the EKG electrode vest 115 can be electrically coupled to the rigid plate 110. The rigid plate 110 can be disposed within the vest 115 (e.g., sewn in or embedded) such that it is located over a particular area of the patient, such as, for example, the patient's sternum, during the medical procedure, as shown in Figure 1. In other embodiments, the rigid plate 110 can be located over any other location of the patient's body.

In some embodiments, the rigid plate 110 can be multiple rigid plates 110. The multiple rigid plates 110 can be placed on multiple places on the patient's body. In some embodiments, each rigid plate 110 can be a unique shape and/or include a unique pattern. In embodiments using multiple rigid plates, each rigid plate 110 can include position sensors, such as, for example, electromagnetic sensors 140, 145. In other embodiments, only some rigid plates 110 include position sensors.

In some embodiments, the rigid plate 110 can include unique markings or the uniquely shaped plate, or the uniquely shaped fiducials 120, 125, 130, 135 can be used in realigning the electrodes 105 used to measure electrogram signals from the body surface after they have been removed. For example, each rigid plate 110 can include markings that enable a medical professional to precisely realign the rigid plate 110 on the patient's body (e.g., the sternum) if the rigid plate 110 is removed from the patient's body. For example, the markings in the rigid plate 110 can be a uniquely shaped cutout, which can allow the medical professional to mark that shape on the patient or the EKG electrode vest 115 before removing the rigid plate 110 from the patient. At a later time, the marking can be aligned with the cutout such that the rigid plate 110 is precisely relocated on the patient. Each rigid plate 110 can include a mechanism for removal and reattachment to the patient's body, such as, for example, a detachable base plate or a sticker that remains affixed to the patient while the rigid plate 110 is removed. Such a removal and reattachment mechanism can aid in re-aligning the rigid plate 110 on the patient at a later time.

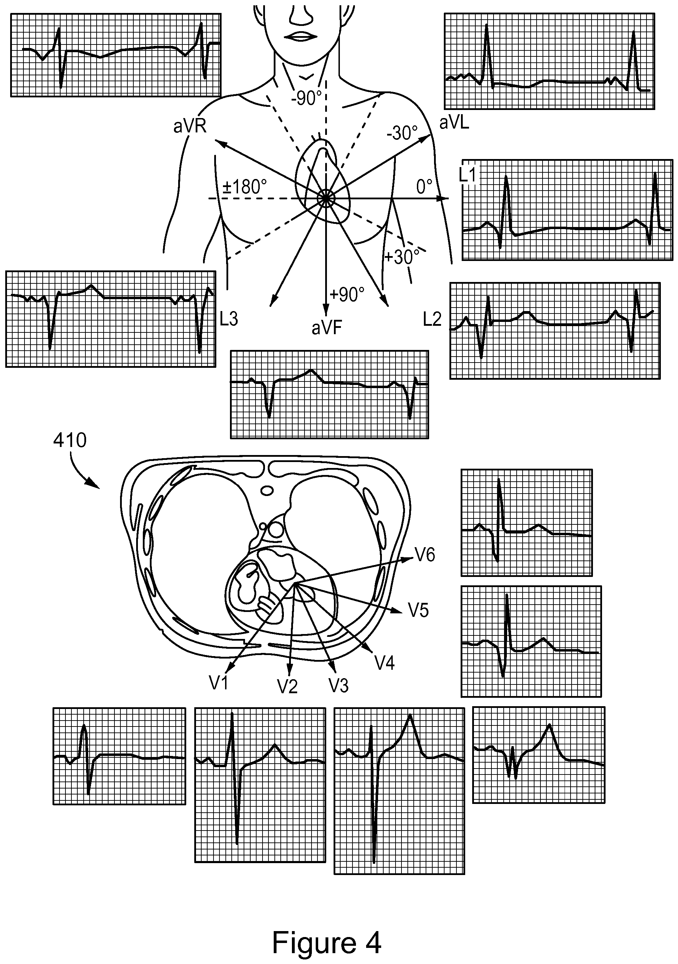

Using the multiple electrodes 105, surface electrograms (EKGs) can be measured. The patient can also be imaged with the multiple electrodes 105 using, for example, Computed Tomography (CT). Using that image data, a model of the heart can be constructed. The cardiac electrogram data that is being measured using the multiple electrodes can be projected onto that model of the heart, as disclosed by U.S. Patent No. 7,983,743 to Rudy, et al, which is incorporated by reference herein in its entirety. Similarly, 12-Lead EKG information can be projected onto a model of the heart as seen in Figure 4 to gain a high-level understanding of the patient heart function.

In some embodiments including multiple rigid plates 110 and/or fiducials 120, 125, 130, 135 that are uniquely shaped, specific fiducials 120, 125, 130, 135 can be segmented out of imagery easily. Specifically shaped fiducials 120, 125, 130, 135 can also aid in correlating the orientation of the fiducials 120, 125, 130, 135 in relation to unique sensors that can be placed on the body. As seen in Figure 1, the two electromagnetic position sensor coils 140, 145 can be positioned orthogonally on the patient. The vector from the center of the triangle fiducial 135 to the square fiducial 120 establishes an axis that is parallel to the orientation of the first electromagnetic position sensor 140 on the body. The vector from the center of the circle fiducial 130 to the center of the square fiducial 120 establishes an axis that is parallel to the orientation of the second electromagnetic position sensor 145 on the body. Once the fiducials 120, 125, 130, 135 are identified in the coordinate system of the image data, the fiducials 120, 125, 130, 135 can enable identification of the location and orientation of each position sensor 140, 145 on the body within the image data coordinate system. When the position sensors 140, 145 are connected to a position sensing system, the volume and coordinate system of the image data can be determined in the coordinate system of the room or position sensing system, also defined as a method of segmentation, correlation, and registration.

In some embodiments, the rigid plate 110 can be electrically coupled to one or more electrodes 105. In such embodiments, the electrodes 105 can drive current through the patient's body to serve as a the basis for a position locating system of instruments within the body as disclosed in U.S. Patent No. 5,983,126 to Wittkampf, et al., which is incorporated herein by reference in its entirety.

In use, the patient can be image scanned (e.g., CT scanned) with the multiple electrodes 105 and the rigid plates 110 applied to the patient. The scan can be displayed on a computer monitor 155, such as, for example, as shown in Figure 1. As shown in the CT scan 155 in Figure 1, the radiopaque fiducials 120, 125, 130, 135 can appear in the CT scan 155. The CT scan 155 in Figure 1 is a two-dimensional image as it is a cross section. In some embodiments, the CT scan 155 can be a stack of two dimensional images (e.g., a CT slice stack, acquired as the CT scanner moves axially from head to toe along the axis of the patient's spine) to create a three-dimensional image such that the fiducials 120, 125, 130, 135 can appear in the image fully as, for example, a circle or rectangle rather the small marks in the CT scan shown in Figure 1. In some embodiments, other types of imaging equipment can be used, such as, for example, X-Ray or MRI.

Software instructions running on a computer that is a part of a position location system can be programmed to receive that image scan data and automatically identify and segment out the unique fiducials 120, 125, 130, 135 associated with respect to the rigid plate 110. Software instructions can be programmed to search for the unique shapes of the fiducials 120, 125, 130, 135 and their geometric orientation in order to determine the location and correlation of position sensors 140, 145 associated with the rigid plate 110 in the coordinate system of the image data (i.e., sensor #1 in position #1 in an image coordinate system and sensor #2 in position #2 in the image coordinate system).

A position tracking system, such as the one disclosed in U.S. Patent Application No. 13/747,266 to Edwards, et al., can be applied to the patient. As shown in Figure 1, an internal reference instrument 160, such as a coronary sinus catheter including a position sensor can be inserted into a stable position within the human heart, such as, for example, the coronary sinus. The electromagnetic sensors 140, 145 coupled to the rigid plate 110 can be connected to the position tracking system. The position tracking system can identify the position of internal sensors and external body surface sensors. The position tracking system can send the signals from the internal sensors and external body surface sensors (i.e., EKG signals and electromagnetic sensor signals 140, 145) to a computer having software instructions for evaluating the signals to identify coordinates for each of the sensors. The coordinates of the sensors within the image data can be used to calculate a transformation of any image data and associated cardiac electrogram data (i.e., body surface electrogram image data) into the coordinate system of the rigid plate 110 and its associated position sensors 140, 145. A transformation matrix can be constructed to apply to any body surface electrogram image data to transform it into the coordinate system of the rigid plate 110. The coordinates of the rigid plate 110 related to its position sensors 140, 145 and the coordinates of the internal reference instrument 160 and its associated position sensors in the coordinate system of the position tracking system can be measured. A transformation matrix can be constructed to apply to any information associated with the rigid plate 110 to transform it into the coordinate system of the internal reference instrument 160. Using the transformation matrices, all body surface electrogram data can be transformed into the coordinate system of the internal reference instrument 160 (e.g., by multiplying the body surface electrogram data by the concatenated transformation matrices). This transformation process can be repeated continuously.

During use, the patient's heart can shift internally with respect to the external body surface rigid plate 110. In such instances, the second transformation matrix can be updated and applied to electrogram data such that it will be projected onto images of the heart accurately. Accuracy can be maintained during minor and major internal changes, such as, for example, if the patient's left lung hyper-inflates during a procedure due to a complication associated with tracheal intubation. As another example, accuracy can be maintained if, for example, the patient's heart shifts with respect to the external electrodes or fiducials.

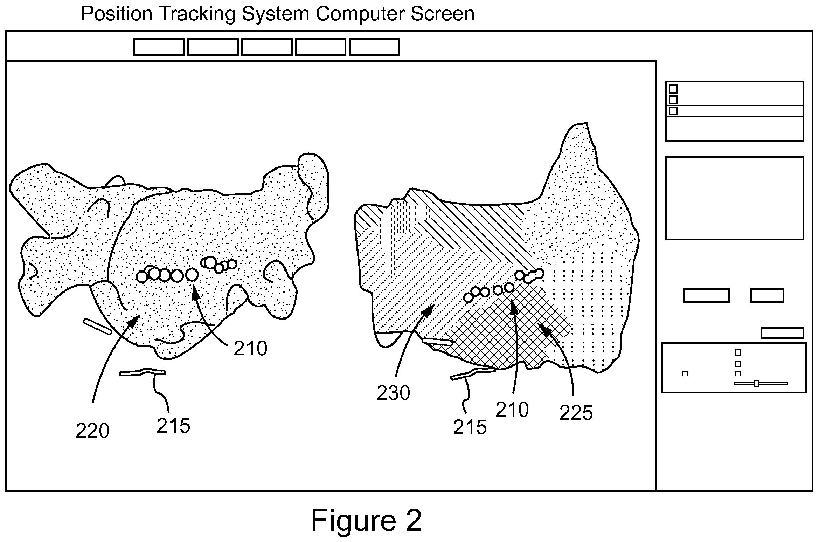

Body surface electrogram information can be automatically registered to a consistent position tracking system and its associated internal reference instrument. The position of other instruments tracked by the tracking system can be displayed in the same consistent tracking system, such as, for example, as depicted in Figure 2.

As shown in Figure 2, a single interface can show the medical professional the positions of electrogram information acquired from the patient's body surface, therapy points 210, intraprocedural annotations, and/or the medical instruments 215 both on an intraprocedurally constructed model of a heart 220 as well as on an image of the patient's heart 230 collected from body surface electrodes. Stated another way, the information obtained from the multiple electrodes 105, rigid plate 110, fiducials 102, 125, 130, 135, position sensors 140, 145, and internal reference instrument 160 can be overlaid on an image of the patient's heart. The image of the patient's heart can be constructed from, for example, the imaging data from, for example, a CT scan 155, MRI, or X-Ray. The transformation process described above can be used to transform all the data into a single coordinate system for display to the user in a unified manner, as shown in Figure 2.

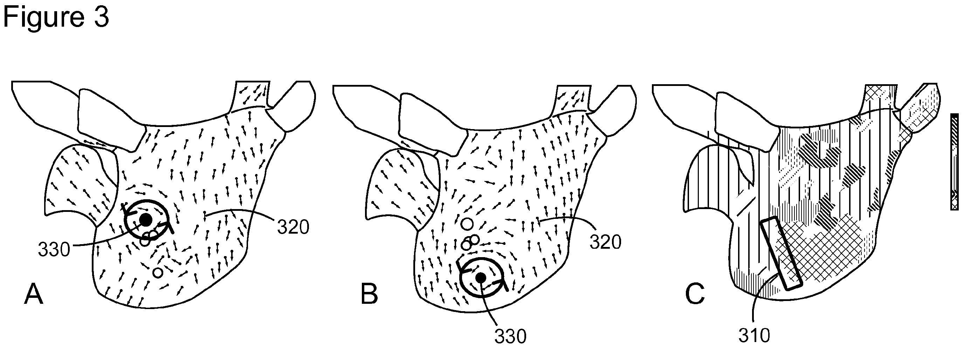

During the course of the procedure, medical instruments such as, for example, a roving ablation catheter, a multi-electrode loop, a multi-electrode star, and/or a multi-electrode basket can be tracked by the position tracking system. The medical instrument can be for example, internal reference instrument 160 of Figure 1. As shown in Figure 3, information can be measured from points within the heart such as voltage 310, temperature, impedance, dominant frequency, conduction velocity 320, force, and hemodynamic pressure.

Figures 3a and 3b are models of a heart showing conduction velocity 320 and potential rotor locations 330. The conduction velocity 320 is information that can be obtained by, for example, internal reference instrument 160 (Figure 1). This information can be sent to a computer for analysis. Similarly, potential rotor location 330 is information that can be obtained by, for example, internal reference instrument 160 (Figure 1). Techniques for collecting and analyzing rotor information, such as the techniques disclosed in U.S. Patent Application No. 14/466,588 to Edwards, et al., hereby incorporated by reference in its entirety, can be used. The information obtained from internal reference instrument 160 can be displayed to the user as an overlay on the model of the heart constructed by the computer, as shown in Figures 3a and 3b.

Figure 3c is similarly a model of a heart showing voltage information. The voltage information can be collected by, for example, internal reference instrument 160 (Figure 1). The information can similarly be overlaid on the model of the heart constructed by the computer, as shown in Figure 3c.

The medical instrument, such as internal reference instrument 160 (Figure 1) can be configured to measure that information and send it to a processor for analysis. This information can be merged on the model of the heart in a co-registered manner with the body-surface electrogram data 225 using the position data from the internal instruments with respect to the internal reference instrument 160 (Figure 1), such as shown in Figure 2. For example, the multiple electrodes 105 (Figure 1) can provide body-surface electrogram data 225 (Figure 2). Also shown in Figure 2 is the model of the heart 220 and image of the heart 225, each from image data obtained from, for example, a CT scan, MRI, or X-Ray, as described elsewhere herein. Further shown in Figure 2 is position information of an internal reference instrument 215, obtained from a position sensor as described elsewhere herein. All of this information can be obtained from different sources and transformed into a single coordinate system for display to the user using the transformation process described above.

The combination of co-registered external and internal based cardiac information can be used in planning therapeutic procedures and augmenting those plans during surgery. Specifically, external body surface electrogram information can be used to identify, annotate, and plan the vicinity of a target location for therapy delivery on a heart image model. During the procedure, various information data such as voltage transitions can be obtained from an internal instrument tracked with respect to the internal reference instrument and co-registered to the external body-surface electrogram information to augment initial treatment plans.

Therapy can be provided by an internal instrument, for example, a roving ablation catheter. In some embodiments, there are multiple internal instruments. For example, the system can include body surface electrodes to collect cardiac electrical activity of the patient from the body surface of the patient as described above. As also described above, an internal reference instrument can be used to collect cardiac electrical activity of the patient from, for example, the coronary sinus. In this way, both internal measurements and external measurements can be collected and used to provide images of the heart that map or show the location of instruments in relation to each other and their location within the patient's body. Further, there can be an internal roving instrument, such as a roving ablation catheter, that can be introduced into the patient's body. The internal roving instrument can include a position sensor to provide the position information to the processor. The processor can convert the position information of the internal roving instrument into any of the coordinate systems already in use such that the location of the internal roving instrument can also be presented with the information of the other instrument information on the model of the heart on the display being used by the surgeon. The surgeon can utilize the information to provide therapy to the patient. For example, a roving ablation catheter can be used to ablate tissue in the patient's heart that may be causing atrial fibrillation. In some embodiments, the internal roving instrument can be configured to provide therapy that creates non-conductive scar tissue.

In some embodiments, for example, body surface electrogram information on a heart model may be used to identify the optimal site to place a pacemaker or defibrillator lead or an entirely miniaturized pacemaker or defibrillator to ensure optimal current flow from the stimulation source through diseased or scarred tissue to stimulate the heart in a manner that resonates with sinus rhythm as opposed to introducing current flow patterns that may cause another unwanted arrhythmia. During the surgery, additional measurements may be taken from a tracked internal instrument to further understand patient characteristics internally, such as voltage transitions indicating scar tissue, and that information can be merged with the original model of the heart and the associated body surface electrogram information. This combined information can be used to tune the output settings of a pacemaker or defibrillator based on external and internal information of the patient. Similar methods of treatment delivery can be constructed for biological drug delivery such as nano-particles, stem-cells, gene therapy.

Once the model of the heart is constructed, an ultrasound probe with its own position sensor capable of being tracked by the position tracking system can be used to create a second model of the heart in the same co-registered coordinate system originating from the body surface electrogram data. The first model of the heart and all associated information can be linearly or non-linearly deformed to mold to the secondary model of the heart. A sound measuring device or set of devices with integrated position sensors can be affixed to the patient's body surface. The speed of sound measurements reaching the external sound measuring devices from a tracked internal ultrasound transducer can be used to augment initial body surface electrogram calculations and associated projections onto a heart model.

Once the data is integrated and co-registered into the coordinate system of the internal reference instrument and its position tracking system, a tracked catheter with one or more magnetic elements may be positioned with the use of high field magnets in order to position that catheter to a target location defined by the co-registered information beginning with the body surface electrogram information.

Once the data is integrated and co-registered into the coordinate system of the internal reference instrument and its position tracking system, a tracked catheter, shrouded by a robotic sheath, may be positioned with the use of robotic sheath manipulator in order to position that catheter to a target location defined by the co-registered information beginning with the body surface electrogram information.

Once the data is integrated and co-registered into the coordinate system of the internal reference and its position tracking system, an external radiation beam source may be positioned with the use of a robotic manipulator in order to position the beam to a target location defined by the co-registered information beginning with the body surface electrogram information.

It is intended that the systems and methods described herein can be performed by software (stored in memory and/or executed on hardware), hardware, or a combination thereof. Hardware modules may include, for example, a general-purpose processor, a field programmable gate array (FPGA), and/or an application specific integrated circuit (ASIC). Software modules (executed on hardware) can be expressed in a variety of software languages (e.g., computer code), including Unix utilities, C, C++, Java^{™}, Ruby, SQL, SAS^{®}, the R programming language/software environment, Visual Basic^{™}, and other object-oriented, procedural, or other programming language and development tools. Examples of computer code include, but are not limited to, microcode or micro-instructions, machine instructions, such as produced by a compiler, code used to produce a web service, and files containing higher-level instructions that are executed by a computer using an interpreter. Additional examples of computer code include, but are not limited to, control signals, encrypted code, and compressed code. Each of the devices described herein can include one or more processors as described above.

Some embodiments described herein relate to devices with a non-transitory computer-readable medium (also can be referred to as a non-transitory processor-readable medium or memory) having instructions or computer code thereon for performing various computer-implemented operations. The computer-readable medium (or processor-readable medium) is non-transitory in the sense that it does not include transitory propagating signals per se (e.g., a propagating electromagnetic wave carrying information on a transmission medium such as space or a cable). The media and computer code (also can be referred to as code) may be those designed and constructed for the specific purpose or purposes. Examples of non-transitory computer-readable media include, but are not limited to: magnetic storage media such as hard disks, floppy disks, and magnetic tape; optical storage media such as Compact Disc/Digital Video Discs (CD/DVDs), Compact Disc-Read Only Memories (CD-ROMs), and holographic devices; magneto-optical storage media such as optical disks; carrier wave signal processing modules; and hardware devices that are specially configured to store and execute program code, such as Application-Specific Integrated Circuits (ASICs), Programmable Logic Devices (PLDs), Read-Only Memory (ROM) and Random-Access Memory (RAM) devices. Other embodiments described herein relate to a computer program product, which can include, for example, the instructions and/or computer code discussed herein.

While the embodiments have been particularly shown and described, it will be understood that various changes in form and details may be made.

## Claims

1. A system, comprising:
a rigid plate (110) configured to be placed on the body of a patient, the rigid plate including a plurality of unique imaging fiducials (120, 125, 130, 135);
an electromagnetic position sensor (140, 145) configured to provide position information of the rigid plate in a first coordinate system;
a plurality of body surface electrodes (105) coupled to the rigid plate, each body surface electrode configured to be placed on the thorax of the patient and further configured to measure cardiac electrical activity information of the patient in a second coordinate system; and
a control unit configured to (1) receive an image scan (155) of the patient, the image scan including the rigid plate and the plurality of body surface electrodes, (2) receive the cardiac electrical activity information from the plurality of body surface electrodes in the second coordinate system, (3) receive position information of the rigid plate from the electromagnetic position sensor in the first coordinate system, (4) use the position information of the rigid plate in the first coordinate system to construct a transformation matrix and (5) use the transformation matrix to transform the cardiac electrical activity information received from the plurality of body surface electrodes from the second coordinate system into the first coordinate system.

2. The system of claim 1, further comprising:
a monitor configured to display image data; and
the control unit further configured to output the image scan and the cardiac electrical activity information in the first coordinate system in image form to the monitor for display.

3. The system of claim 1, wherein the position sensor is a first position sensor and further comprising:
a roving internal instrument having a second electromagnetic position sensor, the roving internal instrument configured to deliver therapy to the patient to create non-conductive scar tissue.

4. The system of claim 1, wherein the electromagnetic position sensor is a first
electromagnetic position sensor and further comprising:
an internal reference instrument configured to be placed internally in the patient;
a second electromagnetic position sensor coupled to the internal reference instrument, the second electromagnetic position sensor configured to provide position information of the internal reference instrument in a third coordinate system; and
wherein the control unit is further configured to receive the position information of the internal reference instrument in the third coordinate system, use the position information of the internal reference instrument to construct a second transformation matrix and use the second transformation matrix to transform the cardiac electrical activity information from the second coordinate system into the third coordinate system.

5. The system of claim 4, further comprising:
a monitor configured to display image data; and
the control unit further configured to output the image scan, the cardiac electrical activity information, and the position information of the internal reference instrument in the third coordinate system in image form to the monitor for display.

6. The system of claim 4, wherein the cardiac electrical activity information is first cardiac
electrical activity information and further comprising:
a second internal instrument having a third electromagnetic position sensor, the second internal instrument configured to measure cardiac electrical activity of the patient to generate second cardiac electrical activity information;
the control unit further configured to transform the second cardiac electrical activity information into the third coordinate system.

7. The system of claim 4, further comprising:
a roving internal instrument having a third electromagnetic position sensor, the roving internal instrument configured to deliver therapy to the patient to create non-conductive scar tissue.

8. The system of claim 6, further comprising:
a monitor configured to display image data; and
the control unit further configured to output the image scan and the overlaid second cardiac electrical activity information and first cardiac electrical activity information in image form to the monitor for display.

9. The system of claim 1, wherein the plurality of body surface electrodes are the plurality of imaging fiducials, each imaging fiducial from the plurality of imaging fiducials being coupled to the rigid plate.

10. The system of claim 1, wherein the electromagnetic position sensor is coupled to the rigid plate and is configured to be repeatably decoupled from the rigid plate and recoupled to the rigid plate.

11. The system of claim 1, wherein the rigid plate is further configured to be repeatably decoupled from the plurality of body surface electrodes and recoupled to the plurality of body surface electrodes.

12. The system of claim 1, wherein the control unit is configured to transform the cardiac electrical activity information into the first coordinate system by multiplying the cardiac electrical activity information by a concatenated transformation matrix.

13. The system of claim 1, wherein the plurality of body surface electrodes are included in an electrocardiogram vest.

14. The system of claim 1, wherein the plurality of body surface electrodes and the rigid plate are included in an electrocardiogram vest.

15. The system of claim 1, wherein each of the plurality of unique imaging fiducials is a unique shape.

16. The system of claim 1, wherein the at least one of the unique imaging fiducials is a void in the rigid plate.

17. The system of claim 1, wherein each of the plurality of unique imaging fiducials is a unique pattern.

## Patentansprüche

1. System, umfassend:
eine starre Platte (110), die dazu ausgelegt ist, auf dem Körper eines Patienten platziert zu werden, wobei die starre Platte eine Vielzahl von eindeutigen Bildgebungsreferenzpunkten (120, 125, 130, 135) enthält; einen elektromagnetischen Positionssensor (140, 145), der dazu ausgelegt ist, Informationen über die Position der starren Platte in einem ersten Koordinatensystem bereitzustellen;
eine Vielzahl von Körperoberflächenelektroden (105), die mit der starren Platte gekoppelt sind, wobei jede Körperoberflächenelektrode dazu ausgelegt ist, auf dem Brustkorb des Patienten angeordnet zu werden, und ferner dazu ausgelegt ist, Informationen über die elektrische Herzaktivität des Patienten in einem zweiten Koordinatensystem zu messen; und
eine Steuereinheit, die dazu ausgelegt ist, (1) einen Bildscan (155) des Patienten zu empfangen, wobei der Bildscan die starre Platte und die Vielzahl von Körperoberflächenelektroden enthält, (2) die Informationen über die elektrische Herzaktivität von der Vielzahl von Körperoberflächenelektroden in dem zweiten Koordinatensystem zu empfangen, (3) Informationen über die Position der starren Platte von dem elektromagnetischen Positionssensor in dem ersten Koordinatensystem zu empfangen, (4) die Informationen über die Position der starren Platte im ersten Koordinatensystem zu verwenden, um eine Transformationsmatrix zu erstellen, und (5) die Transformationsmatrix zu verwenden, um die von der Vielzahl der Körperoberflächenelektroden empfangenen Informationen über die elektrische Herzaktivität aus dem zweiten Koordinatensystem in das erste Koordinatensystem zu transformieren.

2. System gemäß Anspruch 1, ferner umfassend:
einen Monitor, der zum Anzeigen von Bilddaten ausgelegt ist; und
die Steuereinheit, die ferner dazu ausgelegt ist, den Bildscan und die Informationen über die elektrische Herzaktivität im ersten Koordinatensystem in Bildform an den Monitor zur Anzeige auszugeben.

3. System gemäß Anspruch 1, wobei der Positionssensor ein erster Positionssensor ist, und ferner Folgendes umfassend:
ein mobiles internes Instrument mit einem zweiten elektromagnetischen Positionssensor, wobei das mobile interne Instrument dazu ausgelegt ist, eine Therapie an den Patienten abzugeben, um nichtleitendes Narbengewebe zu bilden.

4. System gemäß Anspruch 1, wobei der elektromagnetische Positionssensor ein erster elektromagnetischer Positionssensor ist, und ferner Folgendes umfassend:
ein internes Referenzinstrument, das dazu ausgelegt ist, im Inneren des Patienten platziert zu werden;
einen zweiten elektromagnetischen Positionssensor, der mit dem internen Referenzinstrument gekoppelt ist, wobei der zweite elektromagnetische Positionssensor dazu ausgelegt ist, Informationen über die Position des internen Referenzinstruments in einem dritten Koordinatensystem bereitzustellen; und
wobei die Steuereinheit ferner dazu ausgelegt ist, die Positionsinformationen des internen Referenzinstruments in dem dritten Koordinatensystem zu empfangen, die Positionsinformationen des internen Referenzinstruments zu verwenden, um eine zweite Transformationsmatrix zu erstellen, und die zweite Transformationsmatrix zu verwenden, um die Informationen über die elektrische Herzaktivität aus dem zweiten Koordinatensystem in das dritte Koordinatensystem zu transformieren.

5. System gemäß Anspruch 4, ferner umfassend:
einen Monitor, der zum Anzeigen von Bilddaten ausgelegt ist; und
die Steuereinheit, die ferner dazu ausgelegt ist, den Bildscan, die Informationen über die elektrische Herzaktivität und die Informationen über die Position des internen Referenzinstruments im dritten Koordinatensystem in Bildform an den Monitor zur Anzeige auszugeben.

6. System gemäß Anspruch 4, wobei die Informationen über die elektrische Herzaktivität erste Informationen über die elektrische Herzaktivität sind, und ferner Folgendes umfassend:
ein zweites internes Instrument mit einem dritten elektromagnetischen Positionssensor, wobei das zweite interne Instrument dazu ausgelegt ist, die elektrische Herzaktivität des Patienten zu messen, um zweite Informationen über die elektrische Herzaktivität zu erzeugen;
die Steuereinheit ferner dazu ausgelegt ist, die zweiten Informationen über die elektrische Herzaktivität in das dritte Koordinatensystem zu transformieren.

7. System gemäß Anspruch 4, ferner umfassend:
ein mobiles internes Instrument mit einem dritten elektromagnetischen Positionssensor, wobei das mobile interne Instrument dazu ausgelegt ist, eine Therapie an den Patienten abzugeben, um nichtleitendes Narbengewebe zu bilden.

8. System gemäß Anspruch 6, ferner umfassend:
einen Monitor, der zum Anzeigen von Bilddaten ausgelegt ist; und
die Steuereinheit, die ferner dazu ausgelegt ist, den Bildscan und die überlagerten zweiten Informationen über die elektrische Herzaktivität und die ersten Informationen über die elektrische Herzaktivität in Bildform an den Monitor zur Anzeige auszugeben.

9. System gemäß Anspruch 1, wobei die Vielzahl von Körperoberflächenelektroden die Vielzahl von Bildgebungsreferenzpunkten sind, wobei jeder Bildgebungsreferenzpunkt aus der Vielzahl von Bildgebungsreferenzpunkten mit der starren Platte gekoppelt ist.

10. System gemäß Anspruch 1, wobei der elektromagnetische Positionssensor mit der starren Platte gekoppelt ist und dazu ausgelegt ist, wiederholbar von der starren Platte entkoppelt und wieder mit der starren Platte gekoppelt zu werden.

11. System gemäß Anspruch 1, wobei die starre Platte ferner dazu ausgelegt ist, wiederholbar von der Vielzahl von Körperoberflächenelektroden entkoppelt und wieder mit der Vielzahl von Körperoberflächenelektroden gekoppelt zu werden.

12. System gemäß Anspruch 1, wobei die Steuereinheit dazu ausgelegt ist, die Informationen über die elektrische Herzaktivität in das erste Koordinatensystem zu transformieren, indem die Informationen über die elektrische Herzaktivität mit einer verketteten Transformationsmatrix multipliziert werden.

13. System gemäß Anspruch 1, wobei die Vielzahl von Körperoberflächenelektroden in einer Elektrokardiogrammweste enthalten sind.

14. System gemäß Anspruch 1, wobei die Vielzahl von Körperoberflächenelektroden und die starre Platte in einer Elektrokardiogrammweste enthalten sind.

15. System gemäß Anspruch 1, wobei jeder der Vielzahl von eindeutigen Bildgebungsreferenzpunkten eine eindeutige Form aufweist.

16. System gemäß Anspruch 1, wobei mindestens einer der eindeutigen Bildgebungsreferenzpunkte ein Hohlraum in der starren Platte ist.

17. System gemäß Anspruch 1, wobei jeder der Vielzahl von eindeutigen Bildgebungsreferenzpunkten ein eindeutiges Muster aufweist.

## Revendications

1. Système, comprenant :
une plaque rigide (110) configurée pour être placée sur le corps d'un patient, la plaque rigide incluant une pluralité de repères d'imagerie uniques (120, 125, 130, 135) ;
un capteur de position électromagnétique (140, 145) configuré pour fournir des informations de position de la plaque rigide dans un premier système de coordonnées ; une pluralité d'électrodes de surface corporelle (105) couplées à la plaque rigide, chaque électrode de surface corporelle étant configurée pour être placée sur le thorax du patient et également configurée pour mesurer des informations d'activité électrique cardiaque du patient dans un deuxième système de coordonnées ; et
une unité de commande configurée pour (1) recevoir une image de balayage (155) du patient, l'image de balayage incluant la plaque rigide et la pluralité d'électrodes de surface corporelle, (2) recevoir les informations d'activité électrique cardiaque depuis la pluralité d'électrodes de surface corporelle dans le deuxième système de coordonnées, (3) recevoir des informations de position de la plaque rigide depuis le capteur de position électromagnétique dans le premier système de coordonnées, (4) utiliser les informations de position de la plaque rigide dans le premier système de coordonnées pour construire une matrice de transformation et (5) utiliser la matrice de transformation pour transformer les informations d'activité électrique cardiaque reçues depuis la pluralité d'électrodes de surface corporelle depuis le deuxième système de coordonnées dans le premier système de coordonnées.

2. Système selon la revendication 1, comprenant en outre :
un moniteur configuré pour afficher des données d'image ; et
l'unité de commande également configurée pour délivrer l'image de balayage et les informations d'activité électrique cardiaque dans le premier système de coordonnées sous forme d'image au moniteur pour affichage.

3. Système selon la revendication 1, dans lequel le capteur de position est un premier capteur de position, et comprenant en outre :
un instrument interne itinérant ayant un deuxième capteur de position électromagnétique, l'instrument interne itinérant étant configuré pour délivrer une thérapie au patient pour créer un tissu cicatriciel non conducteur.

4. Système selon la revendication 1, dans lequel le capteur de position électromagnétique est un premier capteur de position électromagnétique, et comprenant en outre :
un instrument interne de référence configuré pour être placé à l'intérieur du corps du patient ;
un deuxième capteur de position électromagnétique couplé à l'instrument interne de référence, le deuxième capteur de position électromagnétique étant configuré pour fournir des informations de position de l'instrument interne de référence dans un troisième système de coordonnées ; et
dans lequel l'unité de commande est également configurée pour recevoir les informations de position de l'instrument interne de référence dans le troisième système de coordonnées, utiliser les informations de position de l'instrument interne de référence pour construire une deuxième matrice de transformation et utiliser la deuxième matrice de transformation pour transformer les informations d'activité électrique cardiaque depuis le deuxième système de coordonnées dans le troisième système de coordonnées.

5. Système selon la revendication 4, comprenant en outre :
un moniteur configuré pour afficher des données d'image ; et
l'unité de commande également configurée pour délivrer l'image de balayage, les informations d'activité électrique cardiaque et les informations de position de l'instrument interne de référence dans le troisième système de coordonnées sous forme d'image au moniteur pour affichage.

6. Système selon la revendication 4, dans lequel les informations d'activité électrique cardiaque sont de premières informations d'activité électrique cardiaque, et comprenant en outre :
un deuxième instrument interne ayant un troisième capteur de position électromagnétique, le deuxième instrument interne étant configuré pour mesurer une activité électrique cardiaque du patient pour générer de deuxièmes informations d'activité électrique cardiaque ;
l'unité de commande également configurée pour transformer les deuxièmes informations d'activité électrique cardiaque dans le troisième système de coordonnées.

7. Système selon la revendication 4, comprenant en outre :
un instrument interne itinérant ayant un troisième capteur de position électromagnétique, l'instrument interne itinérant étant configuré pour délivrer une thérapie au patient pour créer un tissu cicatriciel non conducteur.

8. Système selon la revendication 6, comprenant en outre :
un moniteur configuré pour afficher des données d'image ; et
l'unité de commande également configurée pour délivrer l'image de balayage, et les deuxièmes informations d'activité électrique cardiaque et les premières informations d'activité électrique cardiaque superposées sous forme d'image au moniteur pour affichage.

9. Système selon la revendication 1, dans lequel la pluralité d'électrodes de surface corporelle sont la pluralité de repères d'imagerie, chaque repère d'imagerie issu de la pluralité de repères d'imagerie étant couplé à la plaque rigide.

10. Système selon la revendication 1, dans lequel le capteur de position électromagnétique est couplé à la plaque rigide et est configuré pour être de façon répétée découplé de la plaque rigide et à nouveau couplé à la plaque rigide.

11. Système selon la revendication 1, dans lequel la plaque rigide est également configurée pour être de façon répétée découplée de la pluralité d'électrodes de surface corporelle et à nouveau couplée à la pluralité d'électrodes de surface corporelle.

12. Système selon la revendication 1, dans lequel l'unité de commande est configurée pour transformer les informations d'activité électrique cardiaque dans le premier système de coordonnées en multipliant les informations d'activité électrique cardiaque par une matrice de transformation concaténée.

13. Système selon la revendication 1, dans lequel la pluralité d'électrodes de surface corporelle sont contenues dans un gilet d'électrocardiogramme.

14. Système selon la revendication 1, dans lequel la pluralité d'électrodes de surface corporelle et la plaque rigide sont contenues dans un gilet d'électrocardiogramme.

15. Système selon la revendication 1, dans lequel chaque repère de la pluralité de repères d'imagerie uniques est une forme unique.

16. Système selon la revendication 1, dans lequel l'au moins un des repères d'imagerie uniques est un vide dans la plaque rigide.

17. Système selon la revendication 1, dans lequel chaque repère de la pluralité de repères d'imagerie uniques est un motif unique.
